# FASCICULE DE BREVET EUROPEEN

(11) **EP 1 189 539 B1**
(45) Date de publication et mention de la délivrance du brevet: **10.09.2003**
(21) Numéro de dépôt: 00946005.6
(22) Date de dépôt: 23.06.2000
(51) Int. Cl.: A61B 17/28

(54) **INSTRUMENT DE CHIRURGIE ENDOSCOPIQUE**
INSTRUMENT ZUR ENDOSKOPISCHEN CHIRURGIE
ENDOSCOPIC SURGICAL INSTRUMENT

(30) Priorité: 25.06.1999 FR 9908341
(43) Date de publication de la demande: 27.03.2002
(73) Titulaire: La Précision, 74950 Scionzier (FR)
(72) Inventeur: LANGE, Grégoire, F-74000 Annecy (FR)
(74) Mandataire: Bratel, Gérard
(86) Numéro de dépôt international: FR0001769
(87) Numéro de publication internationale: WO01000095

(56) Documents cités:
- WO-A-98/14124
- DE-A- 2 603 370
- US-A- 5 454 827
- US-A- 5 702 408
- US-A- 5 743 456

## Description

La présente invention concerne un instrument pour la chirurgie endoscopique, qui trouve ses applications principalement dans la laparoscopie (chirurgie endoscopique abdominale) et la thoracoscopie (chirurgie endoscopique du thorax).

Cet instrument de chirurgie endoscopique appartient au genre de ceux comprenant une poignée prolongée par une partie allongée tubulaire, elle-même reliée par une articulation multidirectionnelle à une partie terminale ou distale apte à porter un outil, des moyens de commande étant prévus dans la région de la poignée pour commander l'angulation de la partie terminale, par l'intermédiaire d'une transmission mécanique à fils ou câbles passant à l'intérieur de la partie tubulaire, aussi traversée par un câble central de commande de l'outil.

Un instrument de chirurgie endoscopique de ce genre est connu par la demande de brevet européen EP 0 646 356 A2. Grâce à son extrémité distale articulée, un tel instrument offre au chirurgien l'accès à une plus grande partie du champ opératoire, en permettant de choisir la direction d'approche et en rendant l'intervention plus efficace. Ainsi, le chirurgien s'affranchit des contraintes liées à la chirurgie endoscopique, et notamment le passage par un point fixe (tel que point d'entrée dans l'abdomen).

Toutefois, la réalisation décrite et illustrée dans la demande de brevet européen précitée reste imparfaite, et elle comporte en particulier les inconvénients suivants :

Ainsi, dans la réalisation objet de la demande de brevet européen EP 0 646 356 A2, il n'est pas possible de faire tourner la partie distale de l'instrument autour de son propre axe longitudinal, à partir de l'organe de commande situé sur la poignée. De plus, cet organe de commande est situé dans la partie arrière de la poignée, ce qui apparaît être une position peu ergonomique et pouvant conduire à une imprécision de la commande.

La réalisation du document précité comporte, au moins dans certains modes de réalisation, un croisement des fils de transmission, qui inverse le sens de déplacement de l'extrémité distale de l'instrument par rapport à la commande. De plus, le croisement des fils complique leur trajet, et occasionne des frottements. Ce croisement réduit aussi l'espace libre utilisable pour la commande de l'outil porté par l'instrument, par exemple la commande d'ouverture et de fermeture d'une pince.

Les fils de transmission de l'instrument selon le document EP 0 646 356 A2 étant ancrés sur la rotule d'articulation de la partie distale, le débattement maximum autorisé reste relativement limité.

Par ailleurs, dans l'instrument selon le document précité, il n'est prévu aucun moyen de blocage en position du mécanisme d'orientation, dans une position quelconque à maintenir temporairement ; ainsi, dès que l'on relâche la commande, le mécanisme revient en position de départ (position centrale), sous l'effet de ressorts.

Enfin, l'instrument décrit dans le document EP 0 646 352 A2 n'est pas étanche, mis à part un joint torique placé sur la rotule, et il est difficilement nettoyable.

La présente invention vise à éliminer tous ces inconvénients, en fournissant un instrument de chirurgie endoscopique perfectionné, tant dans sa commande rendue particulièrement ergonomique que dans ses possibilités d'utilisation, tout en garantissant l'étanchéité.

A cet effet, l'invention a essentiellement pour objet un instrument de chirurgie endoscopique, du genre mentionné en introduction, dans lequel :
- les moyens de commande, situés dans la partie avant de la poignée, comprennent une couronne sphérique possédant un centre et un axe, la couronne étant orientable en toutes directions à l'intérieur d'un cône par rapport à son centre, et étant tournante autour de son axe,
- la couronne sphérique est liée en angulation à une pièce de commande des fils ou câbles, à laquelle sont attachées les extrémités proximales des fils ou câbles de transmission,
- des moyens sont prévus pour transmettre la rotation de la couronne sphérique autour de son axe à un élément intérieur monté tournant autour de l'axe de la partie allongée tubulaire, la rotation de cet élément étant transmise, à travers l'articulation précitée, jusqu'à la partie terminale, de manière à orienter cette partie terminale autour de son propre axe longitudinal, et
- des moyens sont prévus pour le blocage non-permanent de l'articulation précitée, dans toute angulation.

De préférence, la couronne sphérique est liée à l'élément intérieur, monté tournant autour de l'axe de la partie allongée tubulaire, par l'intermédiaire d'un mécanisme à cardan.

Ainsi, l'invention propose un instrument de chirurgie endoscopique, caractérisé principalement par un organe de commande unique, dont l'orientation permet d'articuler selon toute angulation la partie distale de l'instrument, et dont la rotation (autour de son propre axe) permet de commander la rotation de cette partie distale autour de son axe longitudinal. La position de cet organe de commande unique, à l'avant de la poignée, est ergonomique et est à l'image des commandes de simple rotation existantes sur les instruments rigides et rectilignes du commerce, habituellement utilisés par les chirurgiens.

Dans une forme de réalisation de l'invention, l'organe de sortie du mécanisme à cardan est accouplé au câble central de commande de l'outil par l'intermédiaire d'un train d'engrenages apte à transmettre le mouvement rotatif de l'extérieur de la partie tubulaire allongée vers l'intérieur de celle-ci, le train d'engrenages comprenant une roue liée en rotation à l'organe de sortie du mécanisme à cardan, une autre roue liée en rotation à une tige centrale liée au câble de commande de l'outil, et des pignons intermédiaires en prise avec les deux roues précitées.

Selon une autre forme de réalisation de l'invention, l'organe de sortie du mécanisme à cardan est accouplé au câble central de commande de l'outil par des moyens magnétiques, en particulier des aimants permanents, avec interposition d'une paroi étanche fixe amagnétique.

Selon encore une autre forme de réalisation de l'invention, l'organe de sortie du mécanisme à cardan est accouplé directement à un élément intérieur tubulaire tournant, entourant le câble de commande de l'outil et formant avec ce dernier un ensemble tournant.

Selon une disposition simple, la couronne sphérique de commande constitue elle-même l'organe de manoeuvre, actionnable par l'utilisateur.

Toutefois, dans une variante plus avantageuse, la couronne sphérique de commande est entourée concentriquement d'une bague extérieure de manoeuvre, à laquelle elle est accouplée en angulation et en rotation par des moyens magnétiques, en particulier des aimants permanents, avec interposition d'une paroi étanche fixe amagnétique. Cette conception de la commande, avec un organe extérieur manipulé par le chirurgien, accouplé à une couronne de commande intérieure, permet une réalisation étanche, le couplage magnétique réalisé au travers de la paroi étanche fixe permettant un positionnement et un déplacement identiques de la couronne sphérique de commande et de la bague de manoeuvre, sans complication mécanique.

Un tel instrument de chirurgie endoscopique étant habituellement pourvu d'une commande séparée pour l'actionnement de l'outil porté par l'instrument, notamment une commande d'ouverture et de fermeture d'une pince par un mouvement de translation de faible amplitude, il est aussi prévu des moyens complémentaires d'étanchéité, sous la forme de manchons flexibles placés dans les parties proximales et distales. Un manchon intérieur d'étanchéité situé en partie distale subissant une torsion lors de la rotation de cette partie distale autour de son axe longitudinal, il convient de limiter cette torsion pour ne pas déchirer le manchon. A cet effet, l'instrument comporte encore, avantageusement, des moyens mécaniques de limitation de la rotation de la partie distale. Dans un mode de réalisation particulier, ces moyens sont constitués par une pièce montée libre en rotation dans la poignée, pièce entraînée en rotation par un premier doigt lié en rotation avec l'élément intérieur recevant le mouvement de rotation de la couronne sphérique, la pièce précitée comprenant elle-même un deuxième doigt venant en butée sur une partie fixe. Un tel mécanisme autorise une rotation sur un peu moins de deux tours complets.

Selon un autre aspect de l'invention, l'articulation multidirectionnelle entre la partie allongée tubulaire et la partie terminale ou distale de l'instrument comprend une rotule intermédiaire libre, apte à décrire un mouvement d'orientation par rapport à une rotule femelle solidaire de l'extrémité avant de la partie allongée tubulaire et aussi par rapport à une autre rotule femelle solidaire de l'extrémité arrière de la partie terminale de l'instrument, toutes ces rotules étant concentriques, et les fils ou câbles de transmission passant à la périphérie de la rotule intermédiaire, leurs extrémités avant étant liées à la partie terminale de l'instrument.

Ainsi, l'instrument dispose d'une rotule intermédiaire double, par exemple avec un débattement de ± 30° environ pour chacune des deux rotules femelles, par rapport à la rotule intermédiaire, de telle sorte que la partie distale peut être orientée sur ± 60° environ, ceci dans toutes les directions de l'espace.

La rotule intermédiaire comporte de préférence un passage diamétral profilé, traversé par le câble flexible central de commande de l'outil porté par l'instrument, de manière à guider ce câble flexible qui, lui-même, positionne la rotule intermédiaire dans l'orientation idéale, correspondant à un angle égal à la moitié de l'angle total entre la partie tubulaire allongée et la partie distale. Le guidage approprié du câble flexible central assure aussi l'application ferme des deux rotules femelles contre la rotule intermédiaire, tout en évitant une variation de longueur de ce câble pendant l'angulation, de manière à éviter toute ouverture ou fermeture intempestive de l'outil, notamment pince.

Avantageusement, les fils ou câbles de transmission ont leurs extrémités arrière ancrées sur un organe intérieur de commande sphérique qui possède un diamètre supérieur à celui de la rotule intermédiaire, de préférence un diamètre égal au double de celui de la rotule intermédiaire ; ainsi, l'angle de débattement donné à l'organe de commande est reproduit au double sur la partie distale : un débattement de l'organe de commande de ± 30°, compatible avec la structure mécanique et avec les exigences ergonomiques, permet une orientation de ± 60° de la partie distale.

Selon un autre aspect de l'invention, l'instrument comporte des moyens de blocage de l'articulation précitée, qui est notamment une articulation à rotule comme défini précédemment, donc des moyens d'immobilisation de la partie terminale ou distale de l'instrument, dans l'angulation donnée par les moyens de commande. Avantageusement, les moyens de blocage non-permanent de l'articulation entre la partie tubulaire allongée et la partie distale de l'instrument comprennent des organes agissant par pincement des fils ou câbles de transmission, ces organes étant situés sur ladite partie tubulaire allongée, de préférence à une distance relativement faible de l'articulation, telle qu'articulation à rotule, ce qui en augmente l'efficacité. En effet, les fils ou câbles devant rester souples pour permettre la commande de l'angulation, il n'est guère approprié de bloquer ces fils ou câbles à proximité de l'organe de commande, car l'allongement des fils ou câbles nuirait au blocage.

Selon un mode de réalisation, les organes de pincement des fils ou câbles de transmission, en vue du blocage de l'articulation précitée, comprennent une bague avec gorges, montée tournante autour de la partie tubulaire allongée et agissant par coincement simultané de tous les fils ou câbles, eux-mêmes positionnés dans des gorges longitudinales du corps principal de la partie allongée tubulaire. Une rotation limitée de la bague, dans un sens choisi, bloque les fils de transmission ou les libère au contraire.

Selon un autre mode de réalisation, les moyens de pincement des fils ou câbles de transmission, en vue du blocage de l'articulation précitée, comprennent une bague ou un manchon de coincement monté coulissant dans la direction axiale de la partie tubulaire allongée, et comportant une partie tronconique agissant sur les fils ou câbles. Cette variante autorise une commande " automatique " du blocage et du déblocage ; en particulier le déblocage se produit dès qu'on agit sur l'organe de commande.

Dans une forme d'exécution particulière, l'ensemble de commande d'angulation et de rotation de la partie distale est mobile en translation suivant l'axe de la partie allongée tubulaire, et permet, par l'intermédiaire d'une came, une commande de coincement/libération des fils ou câbles de transmission, ceci dans le cas d'une bague de pincement rotative telle que précisée ci-dessus.

L'instrument objet de l'invention peut encore comprendre des moyens de blocage par arc-boutement, situés sur la poignée et agissant sur la tige centrale mobile axialement, liée au câble de commande de l'outil, de manière à maintenir la position de cet outil, notamment la fermeture d'une pince.

De toute façon, l'invention sera mieux comprise à l'aide de la description qui suit, en référence au dessin schématique annexé représentant, à titre d'exemples, quelques formes de réalisation de cet instrument de chirurgie endoscopique :
Figure 1 est une vue d'ensemble, en coupe longitudinale, d'un instrument de chirurgie endoscopique conforme à la présente invention, dans une première forme de réalisation ;
Figure 2 est une vue en coupe longitudinale, à plus grande échelle, de la région de la poignée de cet instrument ;
Figure 3 est une vue de détail de la région de l'articulation de cet instrument ;
Figure 4 est une vue d'ensemble, en coupe longitudinale, d'une deuxième forme de réalisation de l'instrument objet de l'invention ;
Figure 5 est une vue en coupe longitudinale, à plus grande échelle, du mécanisme de l'instrument de la figure 4 ;
Figure 6 est une vue partielle, en coupe, d'une variante de l'instrument des figures 4 et 5 ;
Figure 7 est une vue partielle, en coupe, d'une autre variante de cet instrument ;
Figures 8 et 9 sont des vues illustrant, dans deux positions, les moyens de blocage de l'articulation.

L'instrument de chirurgie endoscopique, représenté au dessin, comporte de façon générale, d'arrière en avant : une poignée 1 de préhension et de commande, une partie tubulaire allongée 2 d'axe longitudinal 3, une articulation à rotule 4, une partie terminale ou distale 5 d'axe longitudinal 6, et un outil 7 porté par l'extrémité de la partie distale 5. La poignée 1 comprend une partie de commande de l'outil 7, en particulier de commande d'ouverture/fermeture de cet outil 7 conformé en pince, et un ensemble 8 de commande de l'angulation de la partie distale 5, relativement à la partie tubulaire allongée 2, par l'articulation 4. L'ensemble 8 assure aussi la commande de l'orientation de la partie distale 5, du moins de son outil terminal 7, autour de son propre axe longitudinal 6. Selon des caractéristiques essentielles de l'invention, l'ensemble 8 de commande de l'angulation et de l'orientation de la partie distale 5 est situé dans la partie avant de la poignée 1, et cet ensemble 8 permet, à partir de la manipulation d'un organe de manoeuvre unique, de commander aussi bien l'angulation de la partie distale 5 que l'orientation de cette partie ou du moins de son outil terminal 7.

Cette commande donne lieu à divers modes de réalisation, selon le degré d'étanchéité recherché et les fonctions annexes réalisées, telles que blocage de la partie distale 5 dans l'angulation qui lui est donnée.

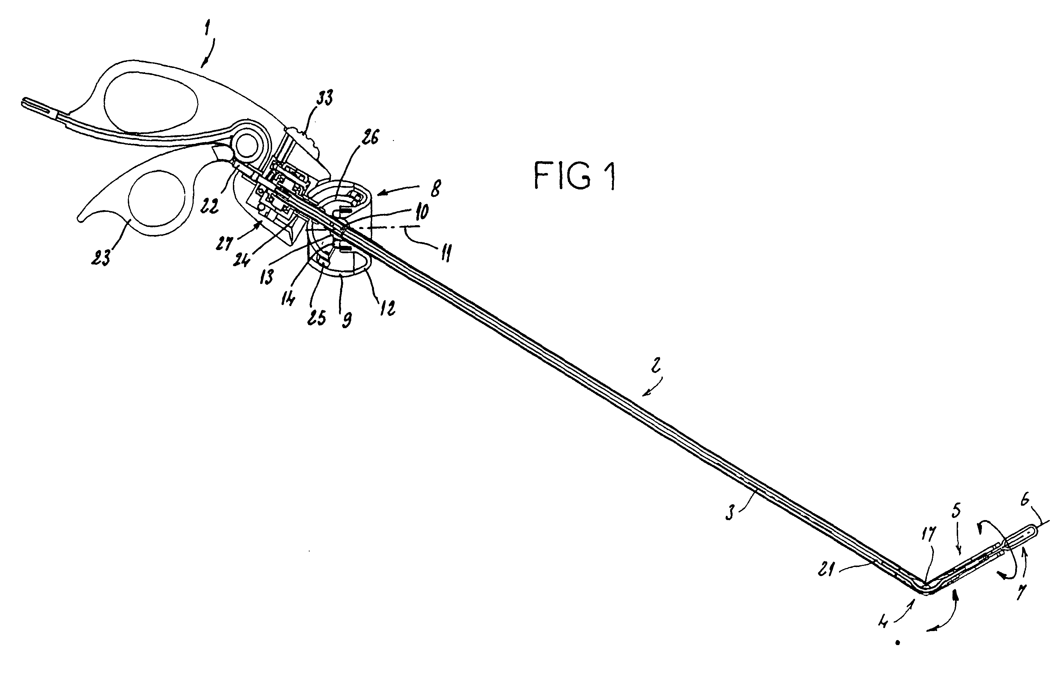

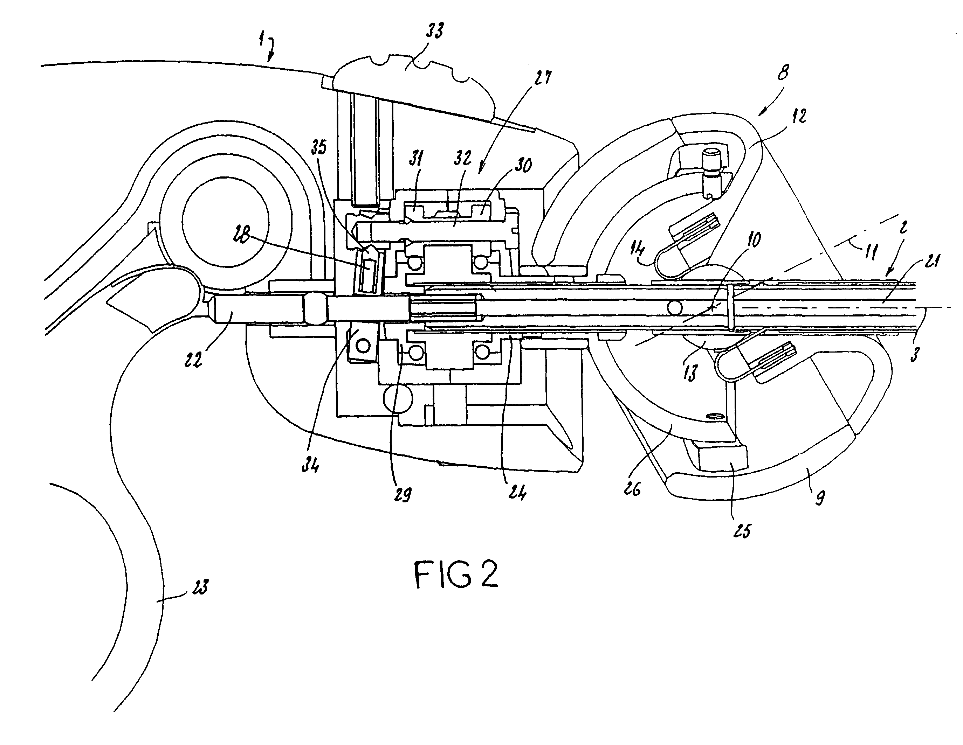

Dans le mode de réalisation des figures 1 à 3, l'ensemble de commande 8 comprend une couronne sphérique 9, dont le centre est amplifié en 10, et l'axe de révolution en 11. La couronne sphérique 9 est complétée, vers l'avant et vers l'intérieur, par une pièce 12 avec dégagement conique, montée autour d'une pièce sphérique centrale 13, traversée par la partie tubulaire allongée 2.

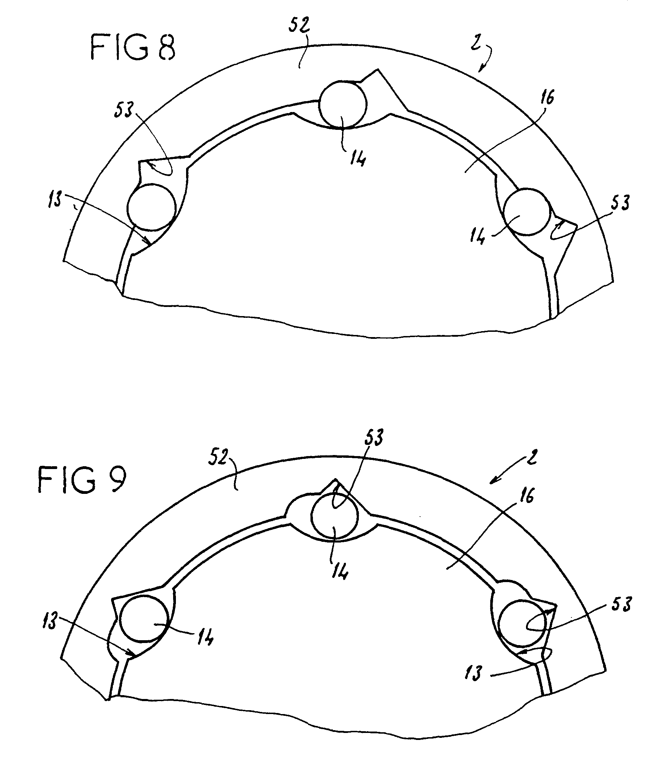

Sur la pièce 12, orientable en toutes directions (à l'intérieur d'un cône) autour du centre 10, sont ancrées les extrémités arrière de câbles de transmission 14, au nombre de six. Les câbles de transmission 14 s'étendent longitudinalement à l'intérieur de la partie tubulaire allongée 2, dans des gorges 15 aménagées à intervalles angulaires réguliers dans le corps principal 16 de cette partie - voir figures 8 et 9.

L'articulation 4 entre la partie tubulaire allongée 2 et la partie distale 6 comprend une rotule intermédiaire libre 17, qui coopère avec deux rotules femelles opposées 18 et 19. La première rotule femelle 18 est solidaire de l'extrémité avant de la partie tubulaire allongée 2. La deuxième rotule femelle 19 est solidaire de l'extrémité arrière de la partie distale 5.

Les six câbles de transmission 14 passent à la périphérie de la rotule intermédiaire 17 et leurs extrémités avant sont liées à la partie distale 5. En fait, pour des raisons de facilité de fabrication, les six câbles 14 ne sont avantageusement que trois câbles, à chacun desquels on fait décrire un demi-tour à l'avant. Le déplacement simultané de ces câbles 14 est commandé par la pièce 12, les câbles ainsi déplacés venant glisser sur la pièce sphérique centrale 13 avant de s'engager dans les gorges 15 précédemment mentionnées. Le diamètre de la pièce sphérique 13 est égal au double du diamètre de la rotule intermédiaire 17 ; ainsi, le débattement angulaire donné à la pièce 12 est reproduit en double sur la partie distale 5 articulée. Par exemple, à un angle maximal de débattement de 30° du côté de l'ensemble de commande 8, il correspond une possibilité d'angulation maximale de 60° de la partie distale 5, par rapport à l'axe 3 de la partie tubulaire allongée 2.

La rotule intermédiaire 17 possède un passage diamétral profilé 20, traversé par un câble flexible 21 qui relie la zone proximale de l'instrument à la partie distale 5, et qui sert à la commande d'ouverture/fermeture de l'outil 7. Le câble flexible 21 est guidé, au niveau de l'articulation 4, par le passage 20 de la rotule intermédiaire, et ainsi ce câble flexible 21 assure lui-même le positionnement de la rotule intermédiaire 17, dans une angulation moyenne entre celle des deux rotules femelles 18 et 19.

L'extrémité arrière du câble flexible 21 est solidaire d'une tige 22 montée mobile axialement dans la poignée 1, et actionnable au moyen d'une gâchette 23 appartenant à cette poignée 1.

La couronne sphérique 9 de l'ensemble de commande 8 est accouplée à un élément intérieur tubulaire 24, monté tournant autour de l'axe 3, par l'intermédiaire d'un mécanisme à cardan comprenant une bague intermédiaire 25, articulée à la couronne 9, et un organe de sortie 26 de forme hémisphérique, articulé à la bague intermédiaire 25 et solidaire de l'élément intérieur 24 précité.

Un train d'engrenages à dentures ou à friction, désigné dans son ensemble par 27, accouple en rotation l'élément 24 avec la tige 22, de manière à transmettre le mouvement de l'extérieur de la partie tubulaire allongée 2 vers l'intérieur de celle-ci. Ce train d'engrenages 27, qui forme un boîtier situé dans la poignée 1 en arrière de l'ensemble de commande 8, comprend une première roue intégrée à l'élément 24, une deuxième roue intégrée à un élément tournant 29 en liaison prismatique avec la tige 22, et des pignons intermédiaires 30, 31 montés en position périphérique, sur des arbres 32 parallèles à l'axe central 3, à la manière de pignons planétaires fixes (trois paires de pignons 30, 31 étant par exemple prévues).

La poignée 1 comprend encore une commande manuelle 33 de blocage/déblocage de la tige 22, donc du câble de commande 21 de l'outil 7, avec dispositif d'arc-boutement. Ce dispositif comprend une pièce pivotante 34, pouvant venir en pression sur la tige 22, et munie d'un pion 35 soumis à la poussée d'un ressort 28, qui le maintient en pression sur une came 36 de profil spécifique, réalisée ici dans le prolongement de l'un des arbres 32 portant les pignons 30, 31. La came 36 possède une partie conique qui correspond à l'arc-boutement, donc au blocage, et une partie droite qui permet le débrayage de ce dispositif.

La rotation de la couronne sphérique 9 autour de son axe 11 provoque, par l'intermédiaire du mécanisme à cardan 25, 26 et du train d'engrenages 27 précédemment décrits, une rotation correspondante de la tige 22 autour de l'axe 3, rotation qui est transmise par le câble flexible 21 jusqu'à la partie distale 5, de manière à orienter l'outil 7 autour de l'axe longitudinal 6 de cette partie distale 5.

L'ensemble de commande 8 est ici prévu déplaçable en translation, sur une faible course, dans la direction de l'axe 3 de la partie tubulaire allongée 2. L'utilisateur devra tirer cet ensemble 8 vers l'arrière, pour libérer les câbles de transmission 14 et autoriser l'angulation, via une came qui transforme le mouvement de translation en rotation.

Enfin, il est à noter que dans l'ensemble de commande 8, la couronne sphérique 9 et la pièce 12 complétant cette couronne ont un mouvement de rotule commun, autour du centre 10, mais seule la couronne 9 est entraînée en rotation autour de l'axe 11.

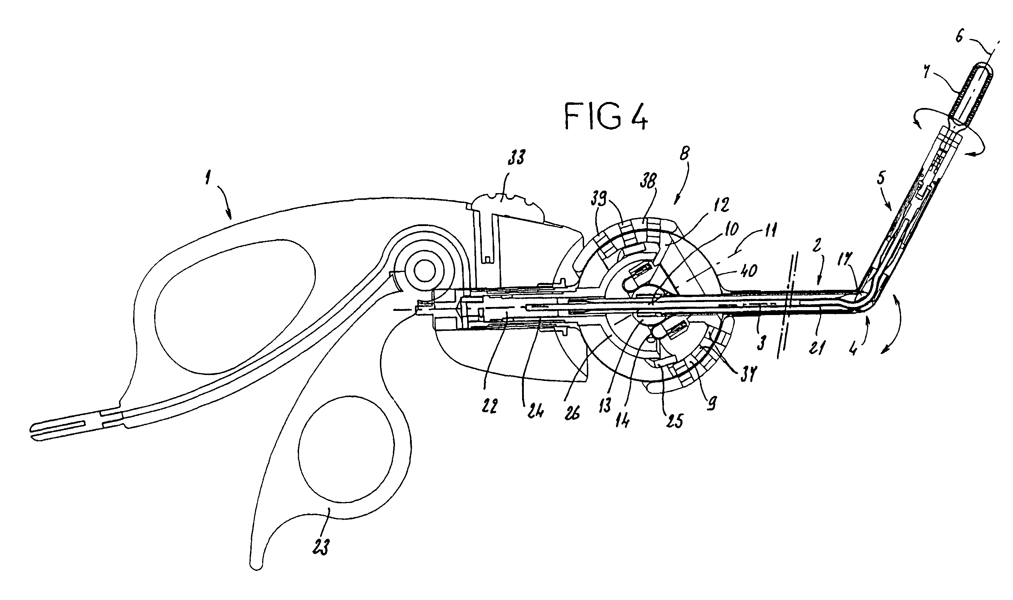

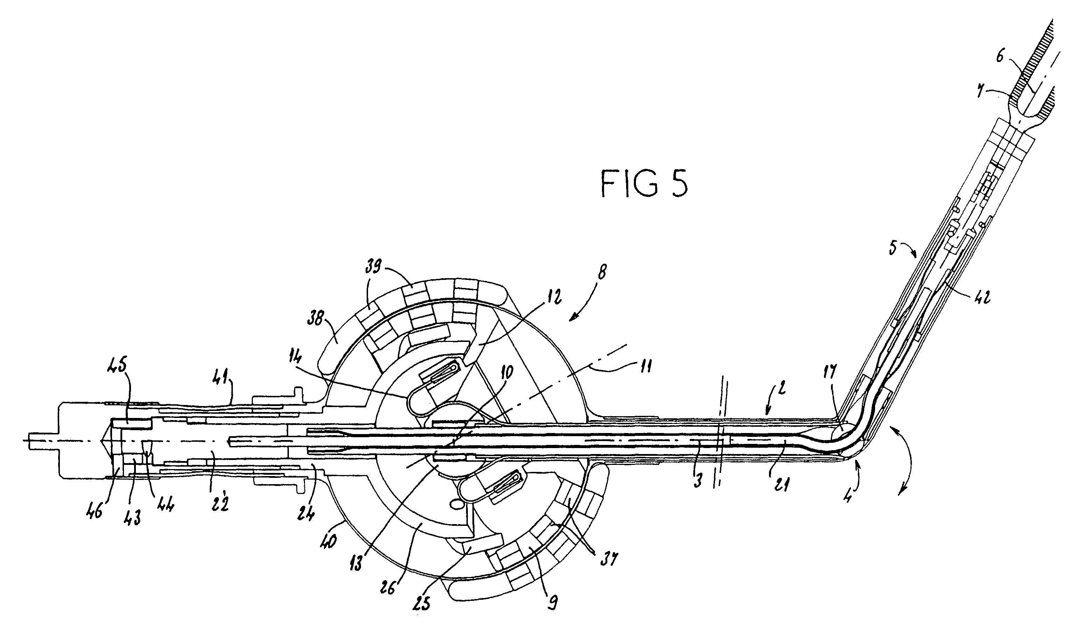

Les figures 4 et 5 représentent une deuxième forme de réalisation de l'instrument. Les éléments communs avec ceux de la forme de réalisation précédente, ou correspondant à ceux-ci sont désignés par les mêmes repères numériques.

En particulier, cette forme de réalisation comprend encore un ensemble de commande 8 avec couronne sphérique 9, orientable autour de son centre 10 et tournante autour de son axe 11, qui permet d'orienter la partie distale 5 et de provoquer la rotation de cette partie distale 5, ou du moins de l'outil terminal 7, autour de l'axe 6. La couronne sphérique 9 est ici solidaire de la pièce 12 à dégagement conique montée autour de la pièce sphérique centrale 13.

Dans la couronne sphérique 9 sont insérés des aimants permanents 37. La couronne sphérique 9 est entourée concentriquement par une bague de manoeuvre extérieure 38, elle aussi conformée en couronne sphérique. Dans la bague de manoeuvre 38 sont insérés d'autres aimants permanents 39, dont le nombre et la disposition correspondent aux aimants 37 de la couronne sphérique 9. Une paroi étanche fixe 40, en matière amagnétique, de forme sphérique, s'étend entre la couronne sphérique 9 et la bague de manoeuvre 38. Ainsi, les aimants 37 et 39 assurent un couplage magnétique entre la bague de manoeuvre 38 et la couronne sphérique 9, à travers la paroi étanche 40, de sorte que les deux pièces supportant des aimants vont se positionner et se déplacer de façon identique, de part et d'autre de la paroi étanche 40.

La couronne sphérique 9, ici actionnée à partir de la bague de manoeuvre 38, commande encore l'angulation de la partie distale 5 par l'intermédiaire des câbles 14, et l'orientation de cette partie distale 5 ou de l'outil 7 par l'intermédiaire d'un mécanisme à cardan 25, 26, comme décrit précédemment. L'organe de sortie 26 du mécanisme à cardan entraîne toutefois ici directement en rotation (c'est-à-dire sans passer par un engrenage) la tige 22, solidaire de l'extrémité arrière du câble 21 de commande de l'outil 7.

La commande d'ouverture/fermeture de l'outil 7 nécessitant ici une petite translation, il est nécessaire de prévoir aussi une étanchéité sur les parties proximale et distale, par des manchons flexibles respectifs 41 et 42. Le manchon 41 situé dans la partie proximale encaisse une simple translation, due à la commande d'ouverture/fermeture de l'outil 7. En revanche, il est nécessaire de limiter la rotation de la partie distale 5 suivant l'axe 6, afin de ne pas déchirer le manchon flexible 42 situé dans la zone distale. Il est prévu à cet effet, au niveau de la poignée 1, une pièce annulaire 43 montée libre en rotation autour de l'axe 3, et entraînée en rotation par un doigt 44 solidaire de la tige 22. La pièce 43 porte un autre doigt 45, venant contre une butée fixe 46. La rotation de la tige 2, donc du câble flexible 21 est ainsi limitée à un peu moins de deux tours.

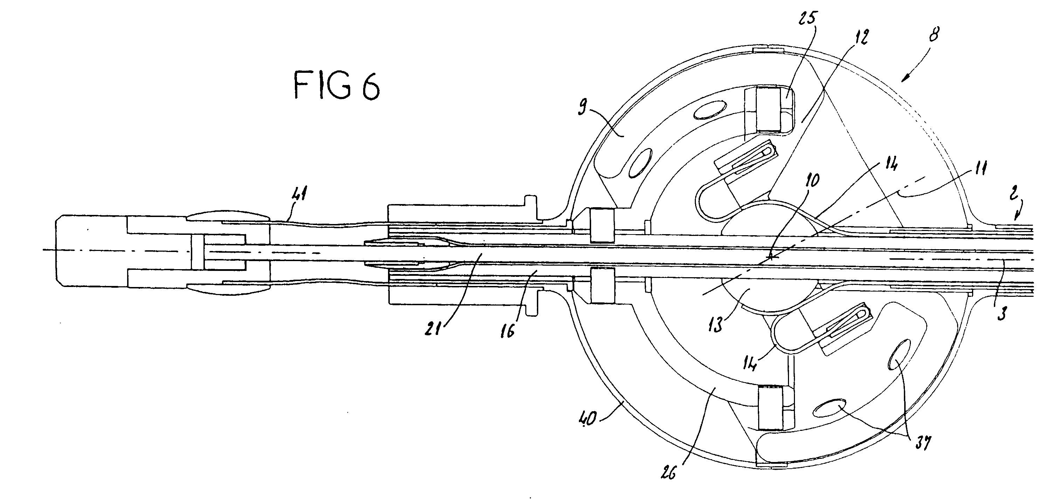

La figure 6 montre une variante de la forme de réalisation précédente, dans laquelle l'organe de sortie 26 du mécanisme à cardan est rendu directement solidaire d'un élément tubulaire intérieur 16, tournant à l'intérieur de la partie tubulaire allongée 2, en même temps que le câble flexible 21 de commande de l'outil 7. Cette réalisation est étanche, comme la précédente, et particulièrement simple.

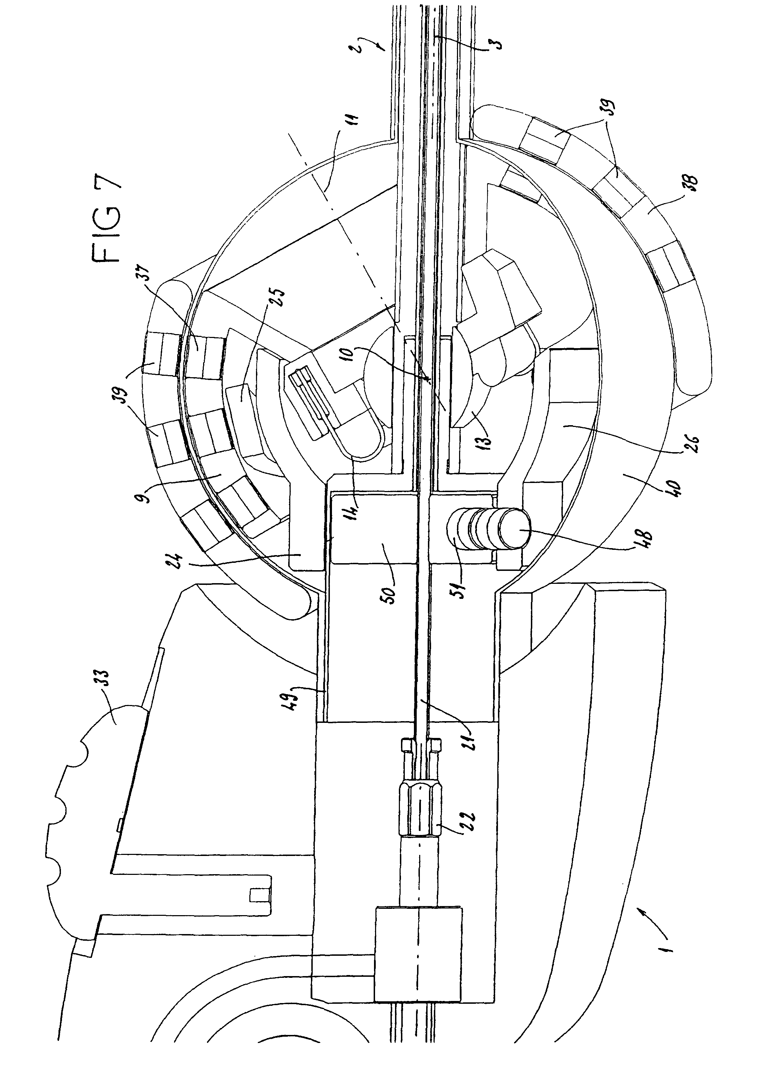

Enfin, la figure 7 montre une autre variante, dans laquelle l'organe de sortie 26 du mécanisme à cardan est accouplé au câble flexible 21 (ou à sa tige terminale 22) par des moyens magnétiques. L'organe de sortie 26 comporte un prolongement tubulaire, dans lequel sont insérés des aimants permanents 48. Une paroi étanche fixe 49 en matière amagnétique est entourée par ledit prolongement tubulaire avec aimants 48. A l'intérieur de la paroi étanche 49 est montée une pièce cylindrique 50, liée en rotation au câble flexible 21, d'autres aimants permanents 51 étant insérés dans la pièce cylindrique 50, leur nombre et disposition correspondant aux aimants 48 de l'organe de sortie 26. Ainsi, les aimants 48 et 51 assurent un couplage magnétique entre l'organe de sortie 26 et la pièce 50, de sorte que les deux pièces occupent à tout moment des positions angulaires correspondantes. Cette dernière forme de réalisation est elle aussi étanche, et même doublement étanche (parois étanches 40 et 49). De plus, une rotation illimitée du câble flexible 21, et par conséquent de l'outil 7, est ici possible.

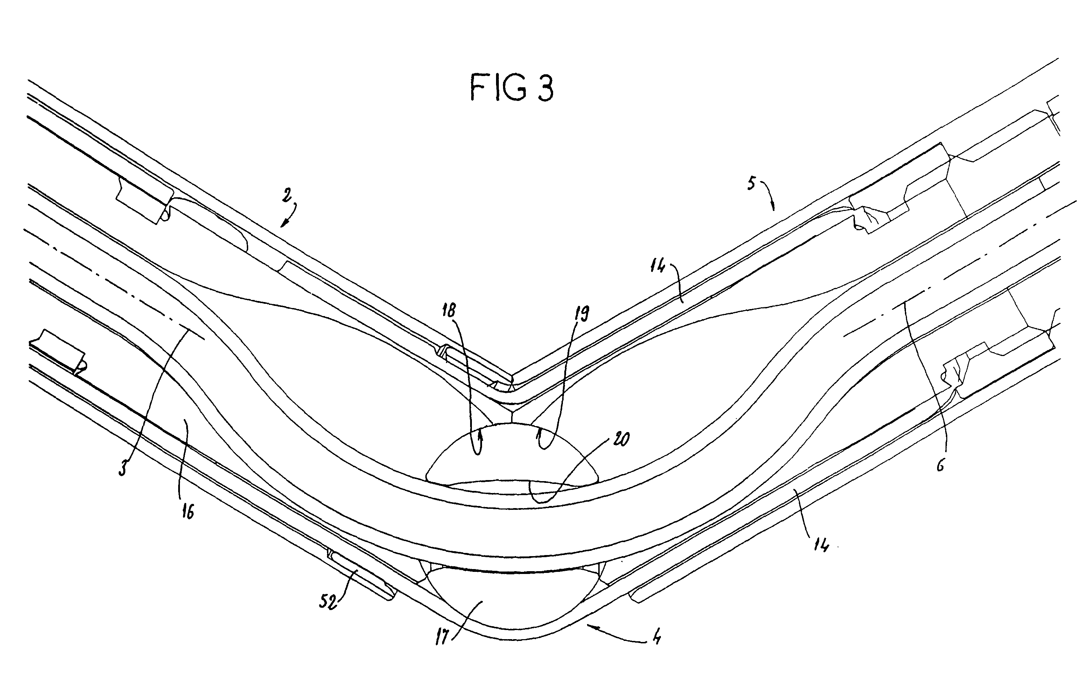

Les figures 8 et 9 illustrent, par ailleurs, une réalisation des moyens qui, par pincement des câbles de transmission 14, permettent de bloquer temporairement la partie distale 5, dans toute orientation donnée à cette partie distale 5 à partir de l'ensemble de commande 8. Comme déjà mentionné plus haut, les câbles 14 passent dans des gorges longitudinales 15 du corps principal 16 de la partie tubulaire allongée 2. Autour de ce corps principal 16, vers l'extrémité de la partie tubulaire 2 voisine de l'articulation 4, est montée tournante une bague de coincement 52 (voir aussi figure 3), elle aussi pourvue de gorges en "V" 53 en correspondance avec les câbles 14. Selon sa position angulaire, la bague 52 bloque les câbles 14 par coincement (figure 8), ou libère ces câbles 14 (figure 9). Le pincement des câbles 14 assure le blocage de l'articulation 4, donc de l'angulation de la partie distale 5. Au contraire, la libération des câbles 14 autorise leur fonction de transmission du mouvement imparti par l'ensemble de commande 8, pour modifier l'angulation de la partie distale 5.

L'on ne s'éloignerait pas du cadre de l'invention, telle que définie dans les revendications annexées :
- en modifiant les détails des divers dispositifs mécaniques ou magnétiques de l'instrument ;
- en remplaçant la bague rotative de coincement des câbles par un manchon de coincement coulissant, agissant sur les câbles par une partie tronconique ;
- en remplaçant la pince terminale par tout autre outil approprié ;
- en prévoyant une commande robotisée et non pas manuelle, sans modifier la conception de l'ensemble de commande, notamment dans une réalisation étanche.

## Revendications

1. Instrument de chirurgie endoscopique, comprenant une poignée (1) prolongée par une partie allongée tubulaire (2), elle-même reliée par une articulation multidirectionnelle (4) à une partie terminale ou distale (5) apte à porter un outil (7), des moyens de commande (8) étant prévus dans la région de la poignée (1) pour commander l'articulation de la partie terminale (5), par l'intermédiaire d'une transmission mécanique à fils ou câbles (14) passant à l'intérieur de la partie tubulaire (2). aussi traversée par un câble central (21) de commande de l'outil (7), **caractérisé en ce que** :
- les moyens de commande (8), situés dans la partie avant de la poignée (1), comprennent une couronne sphérique (9) possédant un centre (10) et un axe (11), la couronne (9) étant orientable en toutes directions à l'intérieur d'un cône par rapport à son centre (10), et étant tournante autour de son axe (11),
- la couronne sphérique (9) est liée en angulation à une pièce de commande (12) des fils ou câbles (14), à laquelle sont attachées les extrémités proximales des fils ou câbles de transmission (14),
- des moyens (25,26) sont prévus pour transmettre la rotation de la couronne sphérique (9) autour de son axe (11) à un élément intérieur (24) monté tournant autour de l'axe (3) de la partie allongée tubulaire (2), la rotation de cet élément (24) étant transmise, à travers l'articulation (4) précitée, jusqu'à la partie terminale (5), de manière à orienter cette partie terminale (5) autour de son propre axe longitudinal (6), et
- des moyens (52,53) sont prévus pour le blocage non-permanent de l'articulation (4) précitée, dans toute angulation.

2. Instrument de chirurgie endoscopique selon la revendication 1, **caractérisé en ce que** la couronne sphérique (9) est liée à l'élément intérieur (24), monté tournant autour de l'axe (3) de la partie allongée tubulaire (2), par l'intermédiaire d'un mécanisme à cardan (25,26).

3. Instrument de chirurgie endoscopique selon la revendication 2, **caractérisé en ce que** l'organe de sortie (26) du mécanisme à cardan est accouplé au câble central (21) de commande de l'outil (7) par l'intermédiaire d'un train d'engrenages (27) apte à transmettre le mouvement de l'extérieur de la partie tubulaire allongée (2) vers l'intérieur de celle-ci, le train d'engrenages (27) comprenant une roue liée en rotation à l'organe de sortie (26) du mécanisme à cardan, une autre roue liée en rotation à une tige centrale (22) liée au câble (21) de commande de l'outil (7) , et des pignons intermédiaires (30,31) en prise avec les deux roues précitées.

4. Instrument de chirurgie endoscopique selon la revendication 2, **caractérisé en ce que** l'organe de sortie (26) du mécanisme à cardan est accouplé au câble central (21) de commande de l'outil (7) par des moyens magnétiques, en particulier des aimants permanents (48,51), avec interposition d'une paroi étanche fixe amagnétique (49).

5. Instrument de chirurgie endoscopique selon la revendication 2, **caractérisé en ce que** l'organe de sortie (26) du mécanisme à cardan est accouplé directement à un élément tubulaire intérieur (16) tournant, entourant le câble (21) de commande de l'outil (7) et formant avec ce dernier un ensemble tournant.

6. Instrument de chirurgie endoscopique selon l'une quelconque des revendications 1 à 5, **caractérisé en ce que** la couronne sphérique (9) de commande constitue elle-même l'organe de manoeuvre actionnable par l'utilisateur.

7. Instrument de chirurgie endoscopique selon l'une quelconque des revendications 1 à 5, **caractérisé en ce que** la couronne sphérique (9) est entourée concentriquement d'une bague extérieure de manoeuvre (38), à laquelle elle est accouplée en angulation et en rotation par des moyens magnétiques, en particulier des aimants permanents (37,39), avec interposition d'une paroi étanche fixe amagnétique (40).

8. Instrument de chirurgie endoscopique selon l'une quelconque des revendications 1 à 7, **caractérisé en ce que** des moyens d'étanchéité sont prévus, sous la forme de manchons flexibles (41,42) placés dans les parties proximale et distale de l'instrument, et **en ce que** des moyens (43 à 46) de limitation de la rotation de la partie distale (5) autour de son axe longitudinal (6) sont prévus.

9. Instrument de chirurgie endoscopique selon la revendication 8, **caractérisé en ce que** les moyens de limitation de la rotation de la partie distale (5) autour de son axe (6) sont constitués par une pièce (43) montée libre en rotation dans la poignée (1), pièce entraînée en rotation par un premier doigt (44) lié en rotation avec l'élément intérieur (24) recevant le mouvement de rotation de la couronne sphérique (9), la pièce (43) précitée comportant elle-même un deuxième doigt (45) venant en butée sur une partie fixe (46).

10. Instrument de chirurgie endoscopique selon l'une quelconque des revendications 1 à 9, **caractérisé en ce que** l'articulation (4) entre la partie allongée tubulaire (2) et la partie terminale ou distale (5) de cet instrument comprend une rotule intermédiaire libre (17), apte à décrire un mouvement d'orientation par rapport à une rotule femelle (18) solidaire de l'extrémité avant de la partie allongée tubulaire (2) et aussi par rapport à une autre rotule femelle (19) solidaire de l'extrémité arrière de la partie terminale (5) de l'instrument, toutes ces rotules (17,18,19) étant concentriques, et les fils ou câbles de transmission (14) passant à la périphérie de la rotule intermédiaire (17), leurs extrémités avant étant liées à la partie terminale (5) de l'instrument.

11. Instrument de chirurgie endoscopique selon la revendication 10, **caractérisé en ce que** chaque rotule femelle (18,19) possède un débattement de ± 30° environ, par rapport à la rotule intermédiaire (17), de sorte que la partie distale (5) peut être orientée sur ± 60° environ, dans toutes les directions de l'espace.

12. Instrument de chirurgie endoscopique selon la revendication 10 ou 11, **caractérisé en ce que** la rotule intermédiaire (17) comporte un passage diamétral profilé (20), traversé par le câble flexible central (21) de commande de l'outil (7) porté par l'instrument, de manière à guider ce câble flexible (21) qui, lui-même, positionne la rotule intermédiaire (17).

13. Instrument de chirurgie endoscopique selon l'une quelconque des revendications 10 à 12, **caractérisé en ce que** les fils ou câbles de transmission (14) ont leurs extrémités arrière ancrées sur un organe intérieur de commande sphérique (13) qui possède un diamètre supérieur à celui de la rotule intermédiaire (17), de préférence un diamètre égal au double de celui de ladite rotule intermédiaire (17).

14. Instrument de chirurgie endoscopique selon l'une quelconque des revendications 1 à 13, **caractérisé en ce que** les moyens de blocage non-permanent de l'articulation (4) entre la partie tubulaire allongée (2) et la partie distale (5) de l'instrument comprennent des organes (52,53) agissant par pincement des fils ou câbles de transmission (14), ces organes (52,53) étant situés sur ladite partie tubulaire allongée (2), de préférence à une distance relativement faible de l'articulation (4), telle qu'articulation à rotule (17).

15. Instrument de chirurgie endoscopique selon la revendication 14, **caractérisé en ce que** les organes de pincement des fils ou câbles de transmission (14), en vue du blocage de l'articulation (4) précitée, comprennent une bague (52) avec gorges (53), montée tournante autour de la partie tubulaire allongée (2) et agissant par coincement simultané de tous les fils ou câbles (14), eux-mêmes positionnés dans des gorges longitudinales (15) du corps principal (16) de la partie allongée tubulaire (2).

16. Instrument de chirurgie endoscopique selon la revendication 14, **caractérisé en ce que** les organes de pincement des fils ou câbles de transmission (14), en vue du blocage de l'articulation précitée, comprennent une bague ou un manchon de coincement monté coulissant dans la direction axiale de la partie tubulaire allongée (2), et comportant une partie tronconique agissant sur les fils ou câbles (14).

17. Instrument de chirurgie endoscopique selon la revendication 15, **caractérisé en ce que** l'ensemble (8) de commande d'orientation de la partie distale (5) est mobile en translation suivant l'axe (3) de la partie allongée tubulaire (2), et permet, par l'intermédiaire d'une came, une commande de coincement/libération des fils ou câbles de transmission (14).

18. Instrument de chirurgie endoscopique selon l'une quelconque des revendications 3 à 17, **caractérisé en ce que** sont prévus des moyens de blocage par arc-boutement (34,35,36), situés sur la poignée (1) et agissant sur la tige centrale (22) mobile axialement, liée au câble de commande (21) de l'outil (7), de manière à maintenir la position de cet outil (7), notamment la fermeture d'une pince.

19. Instrument de chirurgie endoscopique selon l'une quelconque des revendications 1 à 18, **caractérisé en ce qu'**il comporte une commande (8) robotisée.

## Patentansprüche

1. Instrument für die endoskopische Chirurgie, das einen Handgriff (1) umfasst, der durch ein langgezogenes rohrförmiges Teil (2) verlängert ist, das seinerseits durch ein vielseitig ausrichtbares Gelenk (4) mit einem End-Teil oder Fern-Teil (5) verbunden ist, das geeignet ist, ein Werkzeug (7) zu tragen, wobei im Bereich des Handgriffs (1) Steuermittel (8) vorgesehen sind, um das Gelenk des End-Teils (5) mittels einer mechanischen Übertragung durch Drähte oder Kabel (14) zu steuern, die im Innern des rohrförmigen Teils (2) verlaufen, in dem auch ein zentrales Kabel (21) für die Steuerung des Werkzeugs (7) verläuft, **dadurch gekennzeichnet, dass**:
- die Steuermittel (8), die sich im vorderen Teil des Handgriffs (1) befinden, einen sphärischen Kranz (9) umfassen, der ein Zentrum (10) und eine Achse (11) besitzt, wobei der Kranz (9) im Innern eines Kegels in alle Richtungen, bezogen auf sein Zentrum (10), ausrichtbar und um seine Achse (11) drehbar ist,
- der sphärische Kranz (9) winklig zu einem Steuerteil (12) für die Drähte oder Kabel (14) verbunden ist, an dem die nahen Enden der Transmissions-Drähte oder -Kabel (14) befestigt sind,
- Mittel (25, 26) vorgesehen sind, um die Drehung des sphärischen Kranzes (9) um seine Achse (11) auf ein inneres Element (24) zu übertragen, das drehbar um die Achse (3) des langgezogenen rohrförmigen Teils (2) montiert ist; die Drehung dieses Elements (24) wird über das vorgenannte Gelenk (4) bis zum End-Teil (5) in der Weise übertragen, dass dieses End-Teil (5) um seine eigene Längsachse (6) ausgerichtet wird, und
- Mittel (52, 53) vorgesehen sind für die kurzzeitige Blockade des vorgenannten Gelenks (4) in jeder Winkelstellung.

2. Instrument für die endoskopische Chirurgie nach Anspruch 1, **dadurch gekennzeichnet, dass** der sphärische Kranz (9) mit dem inneren Element (24), das drehbar um die Achse (3) des langgezogenen rohrförmigen Teils (2) montiert ist, vermittels eines Kardan-Mechanismus (25, 26) verbunden ist.

3. Instrument für die endoskopische Chirurgie nach Anspruch 2, **dadurch gekennzeichnet, dass** das Ausgangselement (26) des Kardan-Mechanismus mit dem zentralen Kabel (21) zur Steuerung des Werkzeugs (7) gekoppelt ist vermittels eines Getriebes (27), das geeignet ist, die Bewegung von der Außenseite des langgezogenen rohrförmigen Teils (2) nach seiner Innenseite zu übertragen, wobei das Getriebe (27) ein Rad umfasst, das drehbar mit dem Ausgangselement (26) des Kardan-Mechanismus verbunden ist, und ein anderes Rad, das drehbar mit einer zentralen Welle (22) verbunden ist, die mit dem Kabel (21) zur Steuerung des Werkzeugs (7) verbunden ist, sowie Zwischenritzel (30, 31) in Eingriff mit den zwei vorgenannten Rädern.

4. Instrument für die endoskopische Chirurgie nach Anspruch 2, **dadurch gekennzeichnet, dass** das Ausgangselement (26) des Kardan-Mechanismus mit dem zentralen Kabel (21) zur Steuerung des Werkzeugs (7) durch magnetische Mittel gekoppelt ist, insbesondere Permanent-Magnete (48, 51), wobei eine feststehende dichte nichtmagnetische Wand (49) dazwischen eingefügt ist.

5. Instrument für die endoskopische Chirurgie nach Anspruch 2, **dadurch gekennzeichnet, dass** das Ausgangselement (26) des Kardan-Mechanismus direkt mit einen drehenden inneren rohrförmigen Element (16) gekoppelt ist, das das Kabel (21) zur Steuerung des Werkzeugs (7) umgibt und mit diesem letzteren ein drehendes Ensemble bildet.

6. Instrument für die endoskopische Chirurgie nach einem der Ansprüche 1 bis 5, **dadurch gekennzeichnet, dass** der sphärische Steuerungs-Kranz (9) seinerseits das vom Benutzer zu betätigende Steuerteil bildet.

7. Instrument für die endoskopische Chirurgie nach einem dem Ansprüche 1 bis 5, **dadurch gekennzeichnet, dass** der sphärische Kranz (9) konzentrisch von einem äußeren Steuerring (38) umgeben ist, an den er winklig zueinander und drehbar durch magnetische Mittel, insbesondere Permanent-Magnete (37, 39), gekoppelt ist, wobei eine feststehende dichte nichtmagnetische Wand (40) dazwischen eingefügt ist.

8. Instrument für die endoskopische Chirurgie nach einem der Ansprüche 1 bis 7, **dadurch gekennzeichnet, dass** Dichtmittel in Form von flexiblen Manschetten (41, 42) vorgesehen sind die am nahegelegenen und am entfernt liegenden Teil des Instruments angeordnet sind, und dadurch, dass Mittel (43 bis 46) zur Begrenzung der Rotation des Fern-Teils (5) um seine Längsachse (6) vorgesehen sind.

9. Instrument für die endoskopische Chirurgie nach Anspruch 8, **dadurch gekennzeichnet, dass** die Mittel zur Begrenzung der Rotation des Fern-Teils (5) um seine Achse (6) durch ein Element (43) gebildet werden, das frei rotierend im Handgriff (1) montiert ist, ein Element, das durch einen ersten Finger (44) in Rotation versetzt wird, der drehbar mit dem inneren Element (24) verbunden ist, das die Rotationsbewegung des sphärischen Kranzes (9) aufnimmt, wobei das vorgenannte Element (43) seinerseits einen zweiten Finger (45) umfasst, der an einem feststehenden Teil (46) anschlägt.

10. Instrument für die endoskopische Chirurgie nach einem der Ansprüche 1 bis 9, **dadurch gekennzeichnet, dass** das Gelenk (4) zwischen dem langgezogenen rohrförmigen Teil (2) und dem End-Teil oder Fern-Teil (5) dieses Instruments eine freie dazwischenliegende Gelenkkapsel (17) umfasst, die eine Ausrichtbewegung bezogen auf eine Mutter-Gelenkkapsel (18) beschreiben kann, die mit dem vorderen Ende des langgezogenen rohrförmigen Teils (2) fest verbunden ist, und auch bezogen auf eine andere Mutter-Gelenkkapsel (19), die mit dem hinteren Ende des End-Teils (5) des Instruments fest verbunden ist, wobei alle diese Gelenkkapseln (17, 18, 19) konzentrisch zueinander sind, und wobei die Transmissions-Drähte oder -Kabel (14) an der Peripherie der dazwischenliegenden Gelenkkapsel (17) durchlaufen, wobei ihre vorderen Enden mit dem End-Teil (5) des Instruments verbunden sind.

11. Instrument für die endoskopische Chirurgie nach Anspruch 10, **dadurch gekennzeichnet, dass** jede Mutter-Gelenkkapsel (18, 19) einen Bewegungsbereich von etwa ± 30°, bezogen auf die Zwischen-Gelenkkapsel (17), besitzt, so dass das Fern-Teil (5) über etwa ± 60° in alle Richtungen des Raums ausgerichtet werden kann.

12. Instrument für die endoskopische Chirurgie nach Anspruch 10 oder 11, **dadurch gekennzeichnet, dass** die Zwischen-Gelenkkapsel (17) einen diametral verlaufenden profilierten Durchlass (20) umfasst, durch den das flexible zentrale Kabel (21) zur Steuerung des Werkzeugs (7) hindurchläuft, welches von dem Instrument getragen wird, und zwar so, dass dieses flexible Kabel (21) geführt wird, das seinerseits die Zwischen-Gelenkkapsel (17) in Position bringt.

13. Instrument für die endoskopische Chirurgie nach einem der Ansprüche 10 bis 12, **dadurch gekennzeichnet, dass** die Transmissions-Drähte oder -Kabel (14) an ihren hinteren Enden auf einem innenliegenden kugelförmigen Steuerorgan (13) verankert sind, das einen Durchmesser besitzt, der größer ist als derjenige der Zwischen-Gelenkkapsel (17), vorzugsweise einen Durchmesser, der dem doppelten Durchmesser dieser Zwischen-Gelenkkapsel (17) entspricht.

14. Instrument für die endoskopische Chirurgie nach einem der Ansprüche 1 bis 13, **dadurch gekennzeichnet, dass** die Mittel für die kurzzeitige Blockade des Gelenks (4) zwischen dem langgezogenen rohrförmigen Teil (2) und dem Fern-Teil (5) des Instruments Elemente (52, 53) umfassen, die durch Klemmen der Transmissions-Drähte oder -Kabel (14) wirken, wobei diese Elemente (52, 53) sich auf dem langgezogenen rohrförmigen Teil (2) befinden, vorzugsweise in relativ geringem Abstand vom Gelenk (4) wie auch vom Kapselgelenk (17).

15. Instrument für die endoskopische Chirurgie nach Anspruch 14, **dadurch gekennzeichnet, dass** die Elemente zum Klemmen der Transmissions-Drähte oder -Kabel (14) im Hinblick auf die Blockade des vorgenannten Gelenks (4) einen Ring (52) mit Auskehlungen (53) umfassen, der drehbar um den langgezogenen rohrförmigen Teil (2) montiert ist und durch gleichzeitiges Festklemmen aller Drähte oder Kabel (14) wirkt, die ihrerseits in Längs-Auskehlungen (15) des Hauptkörpers (16) des langgezogenen rohrförmigen Teils angeordnet sind.

16. Instrument für die endoskopische Chirurgie nach Anspruch 14, **dadurch gekennzeichnet, dass** die Elemente zum Klemmen der Transmissions-Drähte oder -Kabel (14) im Hinblick auf die Blockade des vorgenannten Gelenks einen Ring oder eine Manschette zum Festklemmen umfassen, der oder die in der axialen Richtung des langgezogenen rohrförmigen Teils (2) gleitend montiert ist und ein kegelstumpfartiges Element enthält, das auf die Drähte oder Kabel (14) einwirkt.

17. Instrument für die endoskopische Chirurgie nach Anspruch 15, **dadurch gekennzeichnet, dass** das Ensemble (8) zur Steuerung der Ausrichtung des Fern-Teils (5) translatorisch entlang der Achse (3) des langgezogenen rohrförmigen Teils (2) beweglich ist und vermittels einer Kurvenscheibe eine Steuerung des Festklemmens / des Lösens der Transmissions-Drähte oder-Kabel (14) erlaubt.

18. Instrument für die endoskopische Chirurgie nach einem der Ansprüche 3 bis 17, **dadurch gekennzeichnet, dass** Mittel (34, 35, 36) zur Blockade durch Stempeldruck vorgesehen sind, die sich am Handgriff (1) befinden und auf die zentrale, axial bewegliche Welle (22) wirken, die mit dem Steuerkabel (21) des Werkzeugs (7) verbunden ist, um so die Position dieses Werkzeugs (7), insbesondere das Schließen einer Klammer, zu bedienen.

19. Instrument für die endoskopische Chirurgie nach einem der Ansprüche 1 bis 18, **dadurch gekennzeichnet, dass** es eine Roboter-Steuerung (8) umfasst.

## Claims

1. Endoscopic surgical instrument, comprising a handle (1) extended by an elongate tubular part (2), itself connected by a multidirectional joint (4) to a terminal or distal part (5) able to carry a tool (7), control means (8) being provided in the area of handle (1) to control articulation of terminal part (5) through a mechanical wire or cable transmission (14) passing inside tubular part (2), also traversed by a central cable (21) controlling tool (7), **characterized in that**:
- the control means (8), located in the front part of handle (1), comprise a spherical crown (9) having a center (10) and an axis (11), crown (9) being orientable in all directions inside a cone relative to its center (10) and being rotatable about its axis (11),
- the spherical crown (9) is linked at an angle to a control part (12) controlling wires or cables (14), the proximal ends of the transmission wires or cables (14) being attached to said control part,
- means (25, 26) are provided to transmit the rotation of the spherical crown (9) about its axis (11) to an internal element (24) mounted to rotate about axis (3) of the elongate tubular part (2), rotation of this element (24) being transmitted, through the aforesaid joint (4), to terminal part (5) in order to orient this terminal part (5) around its own lengthwise axis (6), and
- means (52, 53) are provided for non-permanent locking of the aforesaid joint (4) at any angle.

2. Endoscopic surgical instrument according to Claim 1, **characterized in that** the spherical crown (9) is connected to the internal element (24) mounted to rotate about axis (3) of the elongate tubular part (2) by means of a gimbal mechanism (25, 26).

3. Endoscopic surgical instrument according to Claim 2, **characterized in that** the output element (26) of the gimbal mechanism is coupled to the central cable (21) controlling tool (7) by means of a gear train (27) able to transmit the movement from the outside of the elongate tubular part (2) to the inside thereof, the gear train (27) comprising a gear linked rotationally to the output element (26) of the gimbal mechanism, another gear rotationally linked to a central rod (22) linked to cable (21) controlling tool (7), and intermediate pinions (30, 31) engaging the aforesaid two gears.

4. Endoscopic surgical instrument according to Claim 2, **characterized in that** the output element (26) of the gimbal mechanism is coupled to the central cable (21) controlling tool (7) by magnetic means, in particular permanent magnets (48, 51) with interposition of a fixed nonmagnetic impermeable wall (49).

5. Endoscopic surgical instrument according to Claim 2, **characterized in that** the output element (26) of the gimbal mechanism is coupled directly to an internal rotating tubular element (16) surrounding cable (21) controlling tool (7) and forming a rotating assembly with the latter.

6. Endoscopic surgical instrument according to any one of Claims 1 to 5, **characterized in that** the control spherical crown (9) is itself the operating element activatable by the user.

7. Endoscopic surgical instrument according to any one of Claims 1 to 5, **characterized in that** the spherical crown (9) is surrounded concentrically with an external operating ring (38) to which it is coupled angle- and rotation-wise by magnetic means, in particular permanent magnets (37, 39) with interposition of a fixed nonmagnetic impermeable wall (40).

8. Endoscopic surgical instrument according to any one of Claims 1 to 7, **characterized in that** sealing means are provided in the form of flexible sleeves (41, 42) located in the proximal and distal parts of the instrument, and **in that** means (43 to 46) for limiting the rotation of the distal part (5) about its lengthwise axis (6) are provided.

9. Endoscopic surgical instrument according to Claim 8, **characterized in that** the means for limiting the rotation of distal part (5) about its axis (6) are comprised of a part (43) mounted to rotate freely in handle (1), said part being entrained rotationally by a first finger (44) linked rotationally with the internal element (24) receiving the rotational movement of spherical crown (9), the aforesaid part (43) itself having a second finger (45) that abuts a fixed part (46).

10. Endoscopic surgical instrument according to any one of Claims 1 to 9, **characterized in that** the joint (4) between the elongate tubular part (2) and the terminal or distal part (5) of this instrument comprises a free intermediate ball joint (17) able to describe an orientation movement with respect to a female ball joint (18) integral with the front end of the elongate tubular part (2) and also with respect to another female ball joint (19) integral with the back end of the terminal part (5) of the instrument, all these ball joints (17, 18, 19) being concentric, and the transmission wires or cables (14) passing by the periphery of the intermediate ball joint (17), their front ends being connected to the terminal part (5) of the instrument.

11. Endoscopic surgical instrument according to Claim 10, **characterized in that** each female ball joint (18,19) has a displacement of approximately ± 30° with respect to the intermediate ball joint (17) such that the distal part (5) can be oriented over approximately ± 60° in all directions of space.

12. Endoscopic surgical instrument according to Claim 10 or 11, **characterized in that** the intermediate ball joint (17) has a shaped diametral passage (20) traversed by central flexible cable (21) controlling tool (7) carried by the instrument in order to guide this flexible cable (21) which, itself, positions the intermediate ball joint (17).

13. Endoscopic surgical instrument according to any one of Claims 10 to 12, **characterized in that** the transmission wires or cables (14) have their back ends anchored on an internal spherical control element (13) which has a larger diameter than that of the intermediate ball joint (17), preferably a diameter equal to twice that of intermediate ball joint (17).

14. Endoscopic surgical instrument according to any one of Claims 1 to 13, **characterized in that** the nonpermanent locking means of joint (4) between the elongate tubular part (2) and the distal part (5) of the instrument comprise elements (52, 53) that act by gripping the transmission wires or cables (14), these elements (52, 53) being located on said elongate tubular part (2), preferably at a relatively short distance from joint (4), such as ball joint (17).

15. Endoscopic surgical instrument according to Claim 14, **characterized in that** the elements gripping transmission wires or cables (14) in order to lock the aforesaid joint (4) comprise a ring (52) with recesses (53) mounted to rotate about the elongate tubular part (2) and acting by simultaneously jamming all the wires or cables (14), themselves positioned in lengthwise recesses (15) of the main body (16) of elongate tubular part (2).

16. Endoscopic surgical instrument according to Claim 14, **characterized in that** the elements gripping the transmission wires or cables (14) in order to lock the aforesaid joint comprise a jamming ring or sleeve mounted to slide in the axial direction of the elongate tubular part (2) and having a frustroconical part acting on the wires or cables (14).

17. Endoscopic surgical instrument according to Claim 15, **characterized in that** the assembly (8) controlling the orientation of distal part (5) is translationally movable along the axis (3) of elongate tubular part (2) and, by means of a cam, controls the jamming and release of the transmission wires or cables (14).

18. Endoscopic surgical instrument according to any one of Claims 3 to 17, **characterized in that** buttress type locking means (34, 35, 36) located on handle (1) are provided that act the axially movable central rod (22) that is connected to the control cable (21) of tool (7) in order to maintain the position of this tool (7), particularly the closure of a forceps.

19. Endoscopic surgical instrument according to any one of Claims 1 to 18, **characterized in that** it has robot control (8).
